(19) Europäisches Patentamt
European Patent Office
Office européen des brevets

(11) Veröffentlichungsnummer: **0 062 147**
**A2**

(12) # EUROPÄISCHE PATENTANMELDUNG

(21) Anmeldenummer: **82101057.6**

(22) Anmeldetag: **12.02.82**

(51) Int. Cl.³: **C 07 D 251/64**

(30) Priorität: **31.03.81 DE 3112808**

(43) Veröffentlichungstag der Anmeldung: **13.10.82**
**Patentblatt 82/41**

(84) Benannte Vertragsstaaten: **AT CH DE FR GB IT LI SE**

(71) Anmelder: **CHEMIE LINZ AKTIENGESELLSCHAFT, St. Peter-Strasse 25, A-4020 Linz (AT)**
(84) Benannte Vertragsstaaten: **CH FR GB IT LI SE AT**

(71) Anmelder: **Lentia Gesellschaft mit beschränkter Haftung, Schwanthalerstrasse 39 Postfach 20 16 26, D-8000 München 2 (DE)**
(84) Benannte Vertragsstaaten: **DE**

(72) Erfinder: **Dobramysl, Wilhelm, Dipl.-Ing. Dr., Boschweg 7, A-4020 Linz (AT)**
Erfinder: **Stern, Gerhard, Dipl.-Ing. Dr., Lustenauerstrasse 17, A-4020 Linz (AT)**
Erfinder: **Raml, Walter, Dr., Boschweg 2b, A-4020 Linz (AT)**

(54) **Verfahren zur Herstellung von teilverätherten Methylolmelaminen.**

(57) Verfahren zur Herstellung monomerer, mit aliphatischen Alkoholen mit 1–2 C-Atomen teilverätherter Methylolmelamine mit mindestens 4 Mol Formaldehyd pro Mol Melamin durch Umsetzung von Melamin, Paraformaldehyd und Methanol bzw. Äthanol in einstufiger Reaktion im molaren Verhältnis Melamin zu Formaldehyd zu Alkohol von 1 : 5–10 : 6–20 in praktisch wasserfreiem Medium, unter Stickstoffatmosphäre, bei einem pH-Wert von 7–8, Temperaturen zwischen 50 und 90 °C und einer Reaktionszeit zwischen 3 und 30 Stunden.

EP 0 062 147 A2

0062147

# Verfahren zur Herstellung von teilverätherten Methylolmelaminen

Die Erfindung betrifft ein Verfahren zur Herstellung monomerer, mit Methanol oder Äthanol teilverätherter Methylolmelamine, die pro Mol Melamin mindestens 4 Mole Formaldehyd enthalten,in einer Einstufenreaktion ohne basische Katalyse für die Methylolierung bzw. saure Katalyse für die Verätherung.

Verbindungen dieser Art sind bereits bekannt und haben beispielsweise die nachstehende Formel,

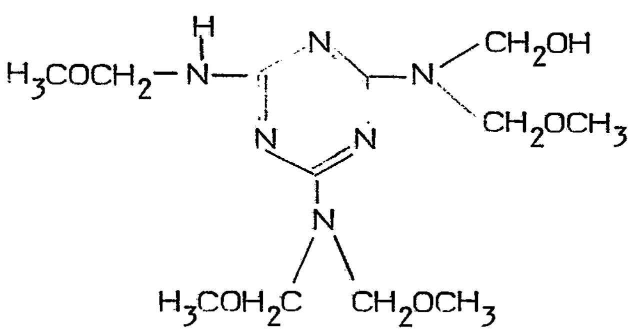

wobei im Durchschnitt auf 1 Mol Melamin 4 - 5,5 Mole Formaldehyd kommen und 1 bis 4 Methylolgruppen veräthert sind. Das heißt, daß sowohl der Methylolierungsgrad als auch der Verätherungsgrad der in Form eines Gemisches erhaltenen Verbindungen verschieden sein kann.

Verbindungen bzw. Mischungen dieser Art werden z.B. in der Lack- oder Textilchemie eingesetzt.

Zur Herstellung dieser Verbindungen sind bereits zahlreiche Verfahren bekannt, bei denen die Methylolierung, vor allem für die Erzielung hoher Methylolierungsgrade, im alkalischen Bereich, die Verätherung jedoch bei sauren pH-Werten durchgeführt wurde, siehe dazu die US-PS 2,529.856, 2,715.619, 2,918.452, die GB-PS 1,030.268, sowie die DE-OS 27 16 006, die DE-OS 28 39 713 und die europäische Patentanmeldung 17.887, wobei gerade in der letztgenannten Patentanmeldung darauf hingewiesen wird, daß in der Verätherungsstufe starke Säuren zugegen sein müssen und dabei schwächeren Säuren vorzuziehen sind. Alle diese Verfahren arbeiten zumindest in der Methylolierungsstufe unter Zusatz von Wasser, das meist in Form von wäßriger Formaldehydlösung oder wäßrigen Laugen in das Reaktionsgemisch eingebracht wird. In der EU-OS 17887 ist u.a. auch ein Arbeiten ohne Zusatz von Wasser vorgesehen, das dann empfohlen wird, wenn niedrige Kondensationsgrade angestrebt werden. Die so erhaltenen verätherten Methylolmelamine haben den Nachteil, daß sie einen beträchtlichen Salzgehalt aufweisen und vielfach auch durch Hydrolyse saure Bestandteile enthalten, die die Lagerstabilität beeinträchtigen, weil dadurch die Weiterkondensation zu Harzen begünstigt wird. Dieser mangelnden Haltbarkeit kann dadurch begegnet werden, daß die Mischungen für die Lagerung alkalisch gemacht werden, was wiederum den Salzgehalt noch mehr erhöht, der für die Verarbeitung z.B. in Lacken stören kann.

Neben diesen zweistufigen Verfahren ist gemäß DE-AS 25 16 349 auch schon ein einstufiges Verfahren zur Herstellung verätherter Methylolmelamine vorgeschlagen worden, gemäß dem Melamin, Formaldehyd und der der Verätherung dienende Alkohol in Gegenwart einer Säure bei einem pH-Wert zwischen 3 und 6,5 unter erhöhtem Druck bei Temperaturen von 80 bis 130°C umgesetzt werden. Der Vorteil dieses Verfahrens soll neben der kurzen Reaktionszeit darin liegen, daß damit auch Produkte mit niedrigeren Methylolierungsgraden erhalten werden. Der Nachteil ist jedoch, daß bei diesem Verfahren schon nach ganz kurzen Reaktionszeiten hohe Kondensationsgrade auftreten, die die Harze beschränkt wasserlöslich oder gar wasserunlöslich machen. Diese Vernetzungstendenz kann nur durch extrem niedere Reaktionszeiten von z.B. nur 1 Minute, eingeschränkt werden. Die ebenfalls zu diesem Zweck empfohlene Maßnahme, wasserfrei zu arbeiten, führt offenbar nicht zum Ziel, wie die

Beispiele erkennen lassen. Zur Vermeidung dieser hohen Vernetzungsgrade ist die gleiche Anmelderin daher wieder zur zweistufigen Arbeitsweise mit alkalischer Methylolierung zurückgekehrt, wie die EU-OS 17887 zeigt.

Der vorliegenden Erfindung lag nun die Aufgabe zugrunde, ein Verfahren zur Herstellung monomerer verätherter Methylolmelamine mit hohem Methylolierungsgrad zu finden, bei dem der störende Salzgehalt der Reaktionsprodukte vermieden und damit deren Lagerstabilität erhöht wird und auch auf die aufwendige 2stufige Verfahrensweise verzichtet werden kann.

Diese Aufgabe konnte überraschenderweise gelöst werden, indem man sowohl Verätherung als auch Kondensation bei neutralem pH und in Abwesenheit von Säure durchführt und das Vorhandensein nennenswerter Mengen an Wasser vermeidet.

Auf diese Weise werden salzfreie Gemische von monomeren verätherten Methylolmelaminen erhalten, die keiner Alkalisierung bedürfen um lagerstabil zu sein.

Gegenstand der Erfindung ist somit ein Verfahren zur Herstellung von monomeren, mit aliphatischen Alkoholen einer C-Anzahl von 1 - 2 teilverätherten Methylolmelaminen, die pro Mol mindestens 4 Mole Formaldehyd enthalten, durch Anlagerung von Formaldehyd an Melamin und gleichzeitiger Verätherung mit den Alkoholen bei erhöhten Temperaturen in einer Einstufenreaktion und anschließendem Verdampfen des überschüssigen Alkohols und Formaldehyds, welches dadurch gekennzeichnet ist, daß man die Reaktionspartner in praktisch wasserfreiem Medium unter Verwendung von Paraformaldehyd in einem molaren Verhältnis Melamin : Formaldehyd : Alkohol von 1 : 5 - 10 : 6 - 20 bei einem pH-Wert von 7 - 8, Temperaturen zwischen 50 und 90°C und Reaktionszeiten zwischen 3 und 30 Stunden in einer Stickstoffatmosphäre miteinander reagieren läßt.

Das bevorzugte Melamin : Formaldehyd : Methanolverhältnis beträgt 1 : 6 - 8 : 8 - 12, die bevorzugten Reaktionstemperaturen liegen zwischen 60 und 80°C und die Reaktionszeiten zwischen 4 und 24 Stunden.

Zur Herstellung werden Melamin, Paraformaldehyd und Methanol bzw. Äthanol vermengt und unter Rühren erwärmt. Die Reihenfolge der Zugabe der Komponenten spielt keine Rolle. Der verwendete Paraformaldehyd enthält vorzugsweise ca. 95 Gew.% Gesamtformaldehyd und soll vorzugsweise auch keine freie Ameisensäure enthalten. Wichtigstes Kriterium ist der pH-Wert der Reaktionslösung, der zwischen 7 und 8 liegen soll und mit dem auch ausgeschlossen wird, daß eine nennenswerte Verunreinigung durch Ameisensäure gegeben ist. Er wird bestimmt, indem eine Probe der Reaktionslösung im Verhältnis 1 : 10 mit Wasser verdünnt wird. Weiters werden, um die Oxidation des Aldehyds durch Luftsauerstoff unter Bildung von Ameisensäure zu verhindern, alle Reaktionen unter einer Stickstoffatmosphäre durchgeführt. Damit wird erreicht, daß keine Säure zugegen ist und daher die Produkte nach Abschluß der Reaktion nicht mit Alkali neutralisiert werden müssen. Somit enthalten die Produkte keine fremden Salze und bedürfen daher auch keiner Alkalisierung zur Erhöhung der Lagerstabilität.

Unter "praktisch wasserfreien Bedingungen" im Sinne der vorliegenden Erfindung ist zu verstehen, daß der Zusatz von Wasser zum Reaktionsgemisch unterbleibt und alle Ausgangsprodukte in konzentrierter Form eingesetzt werden. So werden als Formaldehydkomponente Paraformaldehyd und als Alkoholkomponente reine, durch Destillation wasserfrei gemachte Alkohole eingesetzt. Geringe Mengen Wasser, wie sie in handelsüblichem Paraformaldehyd enthalten sind, können jedoch toleriert werden. Ebenso kann Äthanol 96%ig eingesetzt werden und muß nicht ausdrücklich absolutiert werden.

Für die Steuerung des Methylolierungsgrades (der Methylolierungsgrad ist die durchschnittliche Zahl der angelagerten Mole Formaldehyd, eingesetzt als Paraformaldehyd, pro ein Mol Melamin) ist in erster Linie der Einsatz Molverhältnis Melamin zu Formaldehyd maßgebend. Je größer dieses Verhältnis ist, desto höher ist der Methylolierungsgrad. Bei einem Melamin : Formaldehydverhältnis von 1 : 10 kann ein maximaler Methylolierungsgrad von 5,5 erreicht werden. Höherer Formaldehydeinsatz bewirkt keine wesentliche Steigerung des Methylolierungsgrades. Bei dem bevorzugten Einsatzverhältnis Melamin : Formaldehyd von 1 : 6 - 8 erhält man Methylolierungsgrade um 5,0. Diese Produkte sind ausreichend stabil und zeigen auch noch nach einem Jahr

die erwünschte volle Wasserlöslichkeit. Die untere Grenze des Melamin : Formaldehydverhältnisses liegt bei 1 : 5.

Die Einhaltung des erfindungsgemäßen,praktisch neutralen engen pH-Bereiches zwischen 7 und 8 bringt ferner den Vorteil mit sich, daß die Verätherung optimal abläuft und unerwünschte Vernetzungsreaktionen, die die Wasserlöslichkeit beeinträchtigen, nicht stattfinden.

Die Reaktionszeit und -temperatur spielen für die Methylolierung eine geringere Rolle, dagegen sind diese Parameter für den Verätherungsgrad (das ist die durchschnittliche Anzahl der verätherten Methylolgruppen pro Mol Melamin) bestimmend. Lange Reaktionszeiten und hohe Temperaturen ergeben naturgemäß hohe Verätherungsgrade. Lange Reaktionszeiten kombiniert mit geringer Temperatur bzw. kurze Zeiten und höhere Temperaturen ergeben ähnliche Methylolierungs- und Verätherungsgrade. Das Molverhältnis Melamin : Alkohol ist weniger entscheidend; wichtig ist nur, daß die Suspension rührbar bleibt. Interessanterweise wird die Zeitdauer bis zum Klarpunkt (also bis zu dem Punkt bei dem die Lösung klar wird) durch mehr Alkohol verlängert.

Die Reaktion kann auch bei höherer Temperatur (80 - 90$^0$C) unter Druck durchgeführt werden, wodurch die Reaktionszeiten entsprechend kürzer werden.

Abhängig von den Einsatzverhältnissen und Reaktionsbedingungen kann es vorkommen, daß die Lösungen nicht vollkommen klar werden. Diese schwache Trübung beeinflußt nicht die anwendungstechnischen Eigenschaften, doch kann sie abfiltriert werden, um den optischen Eindruck zu verbessern.

Nach Abschluß der Reaktion wird auf ca. 30 - 40$^0$C gekühlt und bei vermindertem Druck werden die flüchtigen Anteile bis zur gewünschten Konzentration abgezogen. Besonders vorteilhaft läßt sich dieser Vorgang mit einem Dünnschichtverdampfer durchführen. Das wiedergewonnene Lösungsmittel kann ohne Störungen für den nächsten Ansatz verwendet werden.

Die nach der vorliegenden Erfindung gefertigten Produkte zeigen ausgezeichnete anwendungstechnische Eigenschaften. Sie sind in jedem Verhältnis mit Wasser mischbar bzw. löslich. Auf Grund der Herstellungsmethode enthalten sie keine fremden Basen, Säuren und Salze und sind dadurch sehr lagerstabil. Bedingt durch die freien Methylolgruppen sind diese Produkte reaktiver als die vollverätherten Verbindungen. Sie zeigen schon bei tieferen Temperaturen ihre vernetzenden Eigenschaften bzw. sie benötigen dazu keinen Katalysator. Daher sind sie, wie eingangs erwähnt, in den üblichen Anwendungsgebieten von Methylolmelaminen wie z.B. in der Lack- oder Textilchemie vorteilhaft einsetzbar.

Die folgenden Beispiele dienen der näheren Beschreibung der Erfindung. Prozentangaben sind Gewichtsprozente.

Die Bedingungen für das Arbeiten unter Schutzgas und für die Probenvorbereitung wie sie unter Beispiel 1 beschrieben sind, gelten sinngemäß auch für die anderen Beispiele.

Beispiel 1:

In einem 500 ml Vierhals-Kolben mit Rührer, Kühler, Thermometer und Gaseinleitungsrohr werden 40 g Melamin, 73,9 g 95 %iger Paraformaldehyd und 108,5 g Methanol (99 %ig) eingefüllt (pH-Wert des Reaktionsgemisches 7,2, molares Melamin : Formaldehyd : Methanol-Verhältnis 1 : 7,4 : 10,7). Die Apparatur wird mit Stickstoff gespült und während der gesamten Reaktionszeit wird ein schwacher Stickstoffüberdruck aufgegeben. Die Mischung wird über ein Wasserbad auf 65°C erwärmt und unter Rührung 22 Stunden bei dieser Temperatur gehalten. Man erhält eine klare Lösung die auf 40°C abgekühlt wird und auf die gewünschte Konzentration eingeengt wird. Sie kann in dieser Form verwendet werden. Zur Analyse werden 20 g einer 85 % Lösung unter Vakuum zwei Stunden bei 50°C in einem 1 l-Kolben am Rotavapor von flüchtigen Anteilen befreit. Man erhält eine klare Substanz mit 24,5 % Stickstoff und 45,2 % gebundenem Formaldehyd. Das Produkt ist uneingeschränkt mit Wasser verdünnbar. Die $H^1$-NMR Analyse ergibt einen Methylolierungsgrad von 5,0 und einen Verätherungsgrad von 2,9.

Beispiel 2:

Die Umsetzung erfolgt analog zu Beispiel 1 jedoch wird der Ansatz 8 Stunden bei 80°C gehalten und anschließend wiederum auf die gewünschte Konzentration gebracht. Eine kleine Menge wird wiederum am Rotavapor von flüchtigen Anteilen befreit und analysiert. Man erhält ein klares, vollständig wasserverdünnbares Produkt mit 23,6 % Stickstoff und 43,6 % gebundenem Formaldehyd. Die $H^1$-NMR Analyse ergibt einen Methylolierungsgrad von 5,0 und einen Verätherungsgrad von 2,6.

Beispiel 3:

Wie in Beispiel 1 beschrieben, werden 48 g Melamin mit 79 g Paraformaldehyd (95%ig) in 91,5 ml Methanol (99 %ig) umgesetzt (pH-Wert des Reaktionsgemisches 7,9, molares Melamin : Formaldehyd : Methanolverhältnis 1 : 6,6 : 6,0). Die Reaktion wird 3,5 Stunden bei 70°C durchgeführt. Die Lösung wird heiß filtriert, eingeengt und anschließend abgekühlt. Bei Raumtemperatur ist das

Produkt hochviskos und uneingeschränkt wasserverdünnbar. Die Analyse ergibt 25,7 % Stickstoff und 43,7 % gebundenen Formaldehyd. Das $H^1$-NMR Spektrum zeigt einen Methylolierungsgrad von 4,9 und einen Verätherungsgrad von 1,6.

Beispiel 4:

Wie in Beispiel 1 werden 48 g Melamin, 66,2 g Paraformaldehyd (95 %ig) in 134 g Methanol (99 %ig) umgesetzt (pH-Wert des Reaktionsgemisches 7,5, molares Melamin : Formaldehyd : Methanolverhältnis 1 : 5,5 : 11). Das Reaktionsgemisch wird 6,5 Stunden bei 70°C gehalten. Eine stärkere Trübung wird abfiltriert. Nach dem Abziehen des Lösungsmittels unter Vakuum erhält man ein Produkt mit 29,7 % Stickstoff und 42,0 % gebundenem Aldehyd. Das $H^1$-NMR-Spektrum zeigt einen Methylolierungsgrad von 4,2 und einen Verätherungsgrad von 1,2.

Beispiel 5:

Wie in Beispiel 1 werden 48 g Melamin, 114,3 g Paraformaldehyd und 305 ml Methanol entsprechend einem Verhältnis von Melamin : Formaldehyd : Methanol von 1 : 9,5 : 20 umgesetzt, wobei der pH-Wert der Mischung 7,3 beträgt. Dieses Reaktionsgemisch wird 20 Stunden bei 70°C gehalten und ergibt nach dem Eindampfen ein unbeschränkt wasserverdünnbares Produkt mit 22,5 % Stickstoff und 43,0 % gebundenem Formaldehyd. Das $N^1$-NMR Spektrum zeigt einen Methylolierungsgrad von 5,5 und einen Verätherungsgrad von 3,7.

Beispiel 6:

In der Apparatur von Beispiel 1 werden 48 g Melamin, 88,8 g Paraformaldehyd in 189 g Äthanol (96 %ig) bei 80 bis 85°C 6,5 Stunden umgesetzt (pH-Wert des Reaktionsgemisches 7,9, molares Melamin : Formaldehyd : Äthanolverhältnis 1 : 7,4 : 10,7). Nach dem Abziehen des Lösungsmittels enthält man ein Produkt mit 22,1 % Stickstoff und 39,9 % gebundenem Formaldehyd. Die Auswertung des $H^1$-NMR ergibt einen Methylolierungsgrad von 5,1 und einen Verätherungsgrad von 2,1.

Beispiel 7:

In einem 2,5 l Autoklaven mit Rührwerk werden 295 g Melamin, 490 g Paraformaldehyd (95 %ig) und 800 g Methanol (99 %ig) 2,5 Stunden zwischen 70 und 90$^{0}$C gehalten (pH-Wert des Reaktionsgemisches 7,5, molares Melamin : Formaldehyd : Methanolverhältnis 1 : 6,6 : 10,7). Man erhält ein klares farbloses Produkt, das nach dem Abziehen des Lösungsmittels 24,9 % Stickstoff und 45 % gebundenen Formaldehyd enthält. Das $H^1$-NMR zeigt einen Methylolierungsgrad von 5,0 und einen Verätherungsgrad von 2,7.

Beispiel 8:

17 kg Melamin, 28,4 kg Paraformaldehyd (95 %) und 46,5 kg Methanol (99 %ig) werden in einem 100 l Kessel eingefüllt, auf 70$^{0}$C erwärmt und 6 Stunden bei dieser Temperatur gehalten bis Klarlöslichkeit erreicht wird (pH-Wert des Reaktionsgemisches 7,5, molares Melamin : Formaldehyd : Methanolverhältnis 1 : 6,7 : 10,8). Anschließend wird das überschüssige Lösungsmittel bei 48$^{0}$C unter Vakuum bis zur gewünschten Konzentration abdestilliert. Wie im Beispiel 1 beschrieben wird eine kleine Menge am Rotavapor von flüchtigen Anteilen befreit und analysiert. Man erhält ein Produkt mit 25,2 % Stickstoff und 45,4 % gebundenem Aldehyd. Die Substanz ist vollkommen wasserverdünnbar. Die $H^1$-NMR Analyse ergibt einen Methylolierungsgrad von 4,9 und einen Verätherungsgrad von 2,2.

Patentansprüche:

1. Verfahren zur Herstellung von monomeren, mit aliphatischen Alkoholen einer C-Anzahl von 1 - 2 teilverätherten Methylolmelaminen, die pro Mol Melamin mindestens 4 Mole Formaldehyd enthalten, durch Anlagerung von Formaldehyd an Melamin und gleichzeitiger Verätherung mit den Alkoholen bei erhöhten Temperaturen in einer Einstufenreaktion und anschließendem Verdampfen des überschüssigen Alkohols und Formaldehyds, dadurch gekennzeichnet, daß man die Reaktionspartner in praktisch wasserfreiem Medium unter Verwendung von Paraformaldehyd in einem molaren Verhältnis Melamin : Formaldehyd : Alkohol von 1 : 5 - 10 : 6 - 20 bei einem pH-Wert von 7 - 8, Temperaturen zwischen 50 und 90 °C und Reaktionszeiten zwischen 3 und 30 Stunden in einer Stickstoffatmosphäre miteinander reagieren läßt.

2. Verfahren nach Anspruch 1, dadurch gekennzeichnet, daß das Melamin : Formaldehyd : Alkoholverhältnis 1 : 6 - 8 : 8 - 12 beträgt.

3. Verfahren nach den Ansprüchen 1 und 2, dadurch gekennzeichnet, daß die Reaktionstemperatur 60 bis 80 °C beträgt.

4. Verfahren nach den Ansprüchen 1 bis 3, dadurch gekennzeichnet, daß die Reaktionszeiten zwischen 4 und 24 Stunden betragen.

O.Z.697

4. 2.1982